Europäisches Patentamt

European Patent Office

Office européen des brevets

(11) Veröffentlichungsnummer: **0 178 493**
**B1**

# EUROPÄISCHE PATENTSCHRIFT

(45) Veröffentlichungstag der Patentschrift:
08.11.89

(21) Anmeldenummer: 85112029.5

(22) Anmeldetag: 23.09.85

(51) Int. Cl.⁴: **C 07 C 51/48, C 07 C 53/08**

(54) Verfahren zur Extraktion von Carbonsäuren aus verdünnten wässrigen Lösungen.

(30) Priorität: 04.10.84 DE 3436348

(43) Veröffentlichungstag der Anmeldung:
23.04.86 Patentblatt 86/17

(45) Bekanntmachung des Hinweises auf die Patenterteilung:
08.11.89 Patentblatt 89/45

(84) Benannte Vertragsstaaten:
AT BE CH DE FR GB IT LI NL

(56) Entgegenhaltungen:
DE-A- 422 073
FR-A- 866 969
GB-A- 390 185

(73) Patentinhaber: HOECHST AKTIENGESELLSCHAFT,
Postfach 80 03 20, D-6230 Frankfurt am Main 80 (DE)

(72) Erfinder: Wojtech, Bernhard, Dr., Kelkheimer Strasse 67,
D-6232 Bad Soden am Taunus (DE)
Erfinder: Steppich, Walter, Dr., Am Hohen Stein 14,
D-6200 Wiesbaden (DE)
Erfinder: Freudenberger, Dieter, Dr., Nelkenweg 9,
D-6238 Hofheim am Taunus (DE)
Erfinder: Riedel, Knut, Dr., Dr.-Helmen-Weg 6,
D-6238 Hofheim am Taunus (DE)

## Beschreibung

Die Erfindung betrifft ein Verfahren zur Extraktion von Carbonsäuren aus wässrigen Lösungen mit einem Carbonsäuregehalt unter 8 Gew.-%.

Als Lösemittel zur Abtrennung wasserlöslicher Carbonsäuren, insbesondere Essigsäure, aus relativ konzentrierten wässrigen Lösungen mittels Flüssig-Flüssig-Extraktion werden beispielsweise Ester, wie Ethylacetat oder Amylacetat, oder Ketone, wie Cyclohexanon oder Methylisobutylketon, benutzt. Solche konventionellen Lösemittel, deren Extraktionswirkung auf einer verbesserten Solvatation der Carbonsäuren im organischen Medium beruht («physikalische Extraktion»), eignen sich aber nicht für die Abtrennung aus stark verdünnten wässrigen Lösungen (unter 5 Gew.-% Carbonsäure), denn die Verteilungskoeffizienten D der Säuren (D = Säurekonzentration der organischen Phase: Säurekonzentration der wässrigen Phase) sind mit Werten um 1 oder darunter für ein wirtschaftliches Verfahren zu gering.

Demgegenüber sind Extraktionsmittel, die Carbonsäuren durch Anlagerung zu Assoziaten (Komplexen), also durch eine chemische Reaktion binden («chemische Extraktion»), auch bei sehr verdünnten Lösungen, z.B. Abwässern, einsetzbar. Solche Extraktionsmittel enthalten funktionelle polare Gruppen mit Elektronendonatoreigenschaften (z.B. Sauerstoff- oder Stickstoffatome), also «basische» Verbindungen, die sich über den sauren (aktiven) Wasserstoff der Carbonsäuren mit diesen assoziieren. Nach US-PS 3 816 524 sind z.B. geeignet Dialkyl-(alkyl)-phosphonate, Alkyl-(dialkyl)-phosphinate, Trialkylphosphinoxide und Dialkylsulfoxide als polare Extraktionsmittel, verdünnt mit verschiedenen unpolaren Lösemitteln. Von den hier genannten Extraktionsmitteln ist Trioctylphosphinoxid (TOPO) am wirksamsten.

Dessen Extraktionsvermögen für Essigsäure als der wichtigsten zu extrahierenden Carbonsäure ist mit D-Werten um 3 für Lösungen in Wasser ohne Salzzusatz deutlich höher als bei den oben genannten Ketonen und Estern. Dennoch ist er noch nicht hoch genug, um den Extraktionsaufwand ausreichend zu senken. So werden im Gegenstrombetrieb für eine Verdoppelung der Carbonsäurekonzentration im Extrakt bei 99% Ausbeute mehr als 8 theoretische Stufen benötigt. Auch die hohen Kosten für das relativ teure TOPO sind zu berücksichtigen.

Als Alternative zum TOPO werden wegen der geringeren Kosten und der höheren Verteilungskoeffizienten im Journal of Chemical and Engineering Data, Vol. 23, 144 (1978) höhere tertiäre Amine vorgeschlagen. Die höchsten D-Werte lassen sich hier zwar mit Alkoholen und Chloroform als Verdünnungsmitteln erzielen, doch ist bei den Alkoholen die Tendenz zur Veresterung bei der folgenden destillativen Abtrennung von der Essigsäure von Nachteil und beim Chloroform die Umweltbelastung wegen seiner Wasserlöslichkeit und die Korrosivität durch HCl-Abspaltung. Ausserdem stört die Flüchtigkeit der Verdünnungsmittel bei der destillativen Abtrennung der Essigsäure. Daher wird die Verwendung der zwar weniger wirksamen aber auch weniger reaktiven Ketone als Verdünnungsmittel empfohlen.

Es wurde nun gefunden, dass man ein inertes Extraktionsmittel mit ausserordentlich hohem Extraktionsvermögen erhält, wenn man einem Alkylamin mit mindestens 10 C-Atomen ein Phenol oder ein Naphthol zusetzt.

Gegenstand der Erfindung ist ein Verfahren zur Extraktion von Carbonsäuren aus wässrigen Lösungen mit einem Carbonsäuregehalt unter 8 Gew.-%, dadurch gekennzeichnet, dass man als Extraktionsmittel ein Gemisch aus einem Alkylamin mit einer Gesamtkohlenstoffzahl von mindestens 10 und einem Phenol oder Naphthol verwendet, wobei das Molverhältnis Phenol:Amin oder Naphthol:Amin im Bereich von 0,1:1 bis etwa 11:1 liegt. Die Alkylamine können primär, sekundär oder tertiär sein, und der oder die Alkylreste haben insgesamt mindestens 10, vorzugsweise insgesamt 20 bis 50 C-Atome und können geradkettig, cyclisch oder verzweigt sein. Besonders bevorzugt unter den genannten Aminen sind die tertiären, wie Trioctylamin oder Tridecylamin bzw. deren Gemische.

Als zweite Komponente des Extraktionsmittels werden Phenole oder Naphthole eingesetzt. Vorzugsweise verwendet man alkylierte Phenole, wobei der oder die Alkylreste insgesamt 1 bis 40, vorzugsweise insgesamt 5 bis 20 C-Atome haben und geradkettig oder verzweigt sein können. Die Menge des zugesetzten Phenols sollte so bemessen sein, dass neben der im Assoziat mit dem Amin gebundenen Menge keine wesentliche Menge an weiterem (ungebundenem) Phenol vorhanden ist, während ein Aminüberschuss nicht schadet, weil Amin allein bereits mit Carbonsäuren, wenn auch viel schwächer, assoziiert. Beim Nonylphenol, das mit dem Trinonylamin zu einem Assoziat zusammentritt, sollte z.B. der Phenolanteil im Extraktionsmittel 80 Gew.-% nicht wesentlich übersteigen. Vorteilhaft ist ein Anteil von 40 bis 70 Gew.-% Nonylphenol, wobei jedoch auch geringere Anteile noch wirksam sind.

Zur Extraktion nach dem erfindungsgemässen Verfahren eignen sich wasserlösliche gesättigte und ungesättigte Carbonsäuren mit einer oder mehreren Carboxylgruppen. Die Carbonsäuren können auch substituiert sein, vor allem mit sauerstoffhaltigen funktionellen Gruppen. Beispiele für geeignete Säuren sind Essigsäure, Buttersäure, Glykolsäure, Milchsäure, Malonsäure. Besonders wichtig ist die Extraktion von Essigsäure.

Die wässrigen Lösungen dieser Säuren können auch andere gelöste organische oder anorganische Verbindungen und suspendierte Teilchen enthalten, sofern diese die Extraktion nicht stören. Eine vorhergehende extraktive Abtrennung störender organischer Verunreinigungen ist manchmal erforderlich. Das erfindungsgemässe Extraktionsverfahren erlaubt auch die Gewinnung von Säuren bei grosser Verdünnung, insbesondere aus Abwässern, z.B. aus solchen mit einem Essigsäuregehalt um 1 Gew.-% oder darunter.

Die bevorzugten Extraktionsmittel aus alkylierten Phenolen und tertiären Aminen haben wegen der hohen Siedepunkte der Komponenten (z.B. Nonylphenol, KP.: ~ 300 °C, Trinonylamin, Kp.: ~ 350 °C) den Vorteil, dass man niedriger siedende Carbonsäuren (z.B. Essigsäure, Kp.: 118 °C) leichter destillativ abtrennen kann.

Die Extraktion kann beispielsweise kontinuierlich im Gegenstrom in einem mehrstufigen Extraktor erfolgen, wobei infolge der hohen Verteilungskoeffizienten der Extraktionsmittelbedarf, bezogen auf die wässrige Phase, gering ist, sodass das Verhältnis wässrige Phase:organische Phase (Phasenvolumenverhältnis) bis etwa 50:1 betragen kann. Auf diese Weise ist eine starke Anreicherung der Carbonsäuren im Extrakt möglich. Das grosse Extraktionsvermögen des Extraktionsmittels erlaubt es aber auch, einstufig im Rührkessel zu extrahieren, wobei das Verhältnis wässrige Phase:organische Phase bis etwa 10:1 betragen kann.

Die Isolierung der Carbonsäuren aus dem Extrakt auf destillativem Weg ist besonders bei niedrig siedenden Carbonsäuren vorteilhaft. Auch die Möglichkeit zur Rückextraktion in Wasser bei höherer Temperatur besteht. Ebenso kann die Rückextraktion der Carbonsäure mit Alkalilauge unter Salzbildung erfolgen.

Die Erfindung soll anhand folgender Beispiele erläutert werden.

Beispiel 1

Proben einer wässrigen Essigsäure mit einem Säuregehalt von 1,4 Gew.-% wurden in Schüttelgefässen bei Raumtemperatur und verschiedenen Phasenvolumenverhältnissen ins Gleichgewicht gesetzt, einmal mit einem erfindungsgemässen Extraktionsmittel, bestehend aus einer Aminkomponente im Gemisch mit Nonylphenol in verschiedenen Proportionen, und zum Vergleich mit der Aminkomponente allein. Als Aminkomponente wurde ein Gemisch aus 50 Gew.-% Tri-n-octylamin und 50 Gew.-% Tri-n-decylamin genommen. Die Verteilungskoeffizienten sind der nachfolgenden Tabelle 1 zu entnehmen.

Tabelle 1

| Phasen-volumen-WP:OP | Extraktionsmittel | | Essigsäurekonzentra-tion in Gew.-% | | Verteilungs-koeffizient |
|---|---|---|---|---|---|
| | Gew.-% Amine | Gew.-% Nonyl-phenol | OP | WP | |
| 1:1 | 100 | 0 | 0,8311 | 0,7331 | 1,1 |
| 2:1 | 100 | 0 | 1,0900 | 0,9556 | 1,1 |
| 1:1 | 60 | 40 | 1,342 | 0,0127 | 106 |
| 2:1 | 60 | 40 | 2,567 | 0,0576 | 45 |
| 2:1 | 50 | 50 | 2,580 | 0,270 | 96 |
| 2:1 | 40 | 60 | 2,490 | 0,0138 | 180 |
| 1:1 | 30 | 70 | 1,315 | 0,0043 | 306 |
| 2:1 | 30 | 70 | 2,556 | 0,0114 | 224 |
| 2:1 | 20 | 80 | 2,436 | 0,0757 | 32 |

WP = wässrige Phase, OP = organische Phase

Beispiel 2

Proben von wässrigen Lösungen verschiedener Säuren mit einem Säuregehalt von 5,0 Gew.-% wurden in Schüttelzylindern bei Raumtemperatur und einem Phasenvolumenverhältnis wässrige Phase (WP):organische Phase (OP) von 1:1 bzw. 2:1 einmal mit einem erfindungsgemässen Extraktionsmittel, bestehend aus 60 Gew.-% Tri-n-octylamin/Tri-n-decylamin (1:1) und 40 Gew.-% Nonylphenol ins Gleichgewicht gesetzt und zum Vergleich mit dem Gemisch der tertiären Amine allein. Die Verteilungskoeffizienten zeigt Tabelle 2.

Tabelle 2

| Säure | Phasen-volumen verhältnis WP:OP | Extraktionsmittel Gew.-% Amine | Gew.-% Nonyl-phenol | Säurekonzentration Gew.-% OP | WP | Verteilungs-koeffizient |
|---|---|---|---|---|---|---|
| Buttersäure | 1:1 | 100 | 0 | 5,403 | 0,386 | 14 |
| | 1:1 | 60 | 40 | 5,447 | 0,034 | 160 |
| | 2:1 | 100 | 0 | 9,918 | 0,543 | 18 |
| | 2:1 | 60 | 40 | 10,03 | 0,227 | 44 |
| Glykolsäure | 2:1 | 100 | 0 | 3,465 | 3,668 | 0,94 |
| | 2:1 | 60 | 40 | 7,906 | 1,287 | 6,1 |
| Milchsäure | 1:1 | 100 | 0 | 3,018 | 1,427 | 2,1 |
| | 1:1 | 60 | 40 | 4,605 | 0,045 | 102 |
| Malonsäure | 1:1 | 100 | 0 | 5,834 | 0,082 | 71 |
| | 1:1 | 60 | 40 | 5,850 | <0,020 | >290 |

Beispiel 3

Proben einer wässrigen Essigsäure mit einem Säuregehalt von 1,4 Gew.-% wurden in Schüttelzylindern bei Raumtemperatur und einem Phasenvolumenverhältnis wässrige Phase (WP):organische Phase (OP) von 2:1 mit einem Extraktionsmittel, das 40 Gew.-% Phenol ($C_6H_5OH$) bzw. β-Naphthol im Gemisch mit 60 Gew.-% Tri-n-octylamin/Tri-n-decylamin (1:1) enthielt, ins Gleichgewicht gesetzt. Die Verteilungskoeffizienten sind in Tabelle 3 aufgeführt.

Tabelle 3

| Extraktions-mittel | Essigsäurekonzen-tration (Gew.-%) OP | WP | Verteilungs-koeffizient |
|---|---|---|---|
| Amine-Phe-nol | 2,597 | 0,022 | 118 |
| Amine-β-Naphthol | 2,530 | 0,016 | 158 |

Beispiel 4

Ein Abwasser, das 1,35 Gew.-% Essigsäure enthielt, wurde einstufig in einem Rührbehälter bei Raumtemperatur und einem Phasenvolumenverhältnis von 2:1 (wässrige Phase:organische Phase) mit einem Gemisch aus 30 Gew.-% Tri-n-octyl-/Tri-n-decylamin (1:1) und 70 Gew.-% Nonylphenol extrahiert. Die Essigsäurekonzentration in der wässrigen Phase betrug 0,011 Gew.-% und in der organischen Phase 2,47 Gew.-%, das entspricht einer Extraktionsausbeute von 99,3%. Dieser Versuch wurde mit dem gleichen Abwasser unter sonst gleichen Bedingungen wiederholt, nur wurde jetzt als tertiäres Amin 30 Gew.-% Triisoctylamin genommen. Die Extraktionsausbeute betrug 99,0% bei einer Essigsäurekonzentration von 2,46 Gew.-% in der wässrigen Phase und 0,012 Gew.-% in der organischen Phase.

Beispiel 5

In einem 6-stufigen Mischabsetzer wurde Essigsäure aus einem Abwasser mit einem Essigsäuregehalt von 1,4 Gew.-% im kontinuierlichen Gegenstrom bei Raumtemperatur mit einem Gemisch aus 60 Gew.-% Tri-n-octyl-/Tri-n-decylamin (1:1) und 40 Gew.-% Nonylphenol extrahiert. Das Phasenvolumenverhältnis wässrige Phase:organische Phase war 4:1. Die Essigsäurekonzentration in der wässrigen Phase betrug am Ende 0,11% und in der organischen Phase 6,2%, entsprechend einer Extraktionsausbeute von 99,2%. Aus der organischen Phase wurde an-schliessend Essigsäure unter Normaldruck solan-ge abdestilliert, bis eine Sumpftemperatur von 200 °C erreicht war. Dabei wurden 94 Gew.-% der Essigsäure wiedergewonnen.

**Patentansprüche**

1. Verfahren zur Extraktion von Carbonsäuren aus wässrigen Lösungen mit einem Carbonsäuregehalt unter 8 Gew.-%, dadurch gekennzeichnet, dass man als Extraktionsmittel ein Gemisch aus einem aliphatischen Amin mit einer Gesamtkohlenstoffzahl von mindestens 10 und einem Phenol oder Naphthol verwendet, wobei das Molverhältnis Phenol:Amin oder Naphthol:Amin im Bereich von 0,1:1 bis etwa 11:1 liegt.

2. Verfahren nach Anspruch 1, dadurch gekennzeichnet, dass man ein alkyliertes Phenol einsetzt, wobei der oder die Alkylreste insgesamt 1 bis 40 C-Atome haben.

3. Verfahren nach Anspruch 1, dadurch gekennzeichnet, dass man ein alkyliertes Phenol einsetzt, wobei der oder die Alkylreste insgesamt 5 bis 20 C-Atome haben.

4. Verfahren nach einem der Ansprüche 1 bis 3, dadurch gekennzeichnet, dass man ein Amin mit einer Gesamtkohlenstoffzahl von 20 bis 50 einsetzt.

**Revendications**

1. Procédé d'extraction d'acides carboxyliques de solutions aqueuses contenant moins de 8% de ces acides, procédé caractérisé en ce que l'on utilise comme agent d'extraction un mélange d'un alkylamine ayant au total au moins 10 atomes de carbone et d'un phénol ou d'un naphtol dans un rapport molaire phénol:amine ou naphtol:amine compris entre 0,1 et environ 11.

2. Procédé selon la revendication 1, caractérisé en ce que l'on opère avec un alkylphénol dont le ou les radicaux alkyles ont au total de 1 à 40 atomes de carbone.

3. Procédé selon la revendication 1, caractérisé en ce que l'on opère avec un alkylphénol dont le ou les radicaux alkyles ayant au total de 5 à 20 atomes de carbone.

4. Procédé selon l'une quelconque des revendications 1 à 3, caractérisé en ce que l'on opère avec une amine ayant au total de 20 à 50 atomes de carbone.

**Claims**

1. A process for the extraction of carboxylic acids from aqueous solutions with a carboxylic acid content below 8% by weight, wherein a mixture of an aliphatic amine with a total carbon number of at least 10 and a phenol or naphthol is used as the extracting agent, the molar ratio of phenol:amine or naphthol:amine being in the range from 0,1:1 to around 11:1.

2. A process as claimed in claim 1, wherein an alkylated phenol is used, the alkyl radical or radicals having in total 1 to 40 C atoms.

3. A process as claimed in one of claim 1, wherein an alkylated phenol is used, the alkyl radical or radicals having in total 5 to 20 C atoms.

4. A process as claimed in one of claims 1 to 3, wherein an amine with a total carbon number of 20 to 50 is used.